# EUROPEAN PATENT SPECIFICATION

(11) **EP 2 865 371 B1**
(45) Date of publication and mention of the grant of the patent: **10.08.2016**
(21) Application number: 13189655.7
(22) Date of filing: 22.10.2013
(51) Int. Cl.: A61K 9/16, A61K 31/225, A61K 9/00

(54) **Fast dissolving granules**
Schnelllösliche Granulate
Granules à dissolution rapide

(43) Date of publication of application: 29.04.2015
(73) Proprietor: Disphar International B.V., 7255 PZ Hengelo (NL)
(72) Inventor: Velada, José Luis, 3826 BA Amersfoort (NL); Doshi, Hiteshkumar Anilkant, 400080 Mumbai (IN); Jadha, Vilas, 400080 Mumbai (IN); Banode, Vikrant, 400080 Mumbai (IN)
(74) Representative: Swarte, Veronica Regina

(56) References cited:
- WO-A1-2007/057924
- WO-A2-2009/016608
- WO-A2-2009/034409
- US-A- 2 992 970

## Description

### Field of the invention

This invention relates to fast dissolving granules of sulfosuccinic acid bis(2-ethylhexyl) ester or a salt or solvate thereof and a sugar alcohol.

### Background of the invention

Sulfosuccinic acid bis(2-ethylhexyl) ester and their salts, for example dioctyl sodium sulfosuccinate or docusate sodium (also referred to as DSS), are widely known to be used in enema formulations. They are available in aqueous solutions together with sugar alcohol and are conveniently administered rectally. US 2992970 discloses the use of a potassium salt of sulfosuccinic acid bis(2-ethylhexyl) ester in an aqueous sorbitol solution effective in bowel evacuation. Aqueous enema solutions that contain sulfosuccinic acid bis(2-ethylhexyl) ester salts and sugar alcohol are, however, known to have poor shelf life, apparently due to the instability of the ingredients in aqueous medium. WO 2000/071993 discloses an aqueous solution of sulfosuccinic acid bis(2-ethylhexyl) ester and sugar alcohol in which the shelf life is prolonged by use of a benzoate preservative. Additionally, it is disclosed here that due to the low solubility of sulfosuccinic acid bis(2-ethylhexyl) ester in water, complete dissolution of the ingredients is achieved only after prolonged stirring in aqueous medium. Further, since benzoates are known to be irritants of mucosal lining it use is preferably avoided. In view of volume and weight considerations, liquid formulations are uneconomical to transport. The challenges involved in preparing dry formulations for bowel evacuation include preparing physically stable dosage forms that are capable of disintegrating quickly in aqueous medium and form an aqueous solution that is free from suspended particles. Usually, such formulations are prepared by optimal use of excipients such as diluents, binding agents, stabilizing agents and disintegrants along with the active ingredients. Often, the active ingredients are mixed together in a melt. From the perspective of safety and from the perspective of ease of formulation, it is desirable that, in such dry formulations, the excipients are used in minimal quantities. Mixing the ingredients in a melt is process intensive and often require selective use of excipients. There is, therefore, a need for a formulation of sulfosuccinic acid bis(2-ethylhexyl) ester and sorbitol that overcomes the above mentioned disadvantages.

### Summary of the Invention

The invention provides fast dissolving granules comprising sulfosuccinic acid bis(2-ethylhexyl) ester, a salt or a solvate thereof as a first active ingredient and sorbitol as a second active ingredient, wherein sulfosuccinic acid bis(2-ethylhexyl) ester is present in an amount of 0.2 to 4 percentage by weight of sorbitol.
In one embodiment, the invention provides fast dissolving granules comprising sulfosuccinic acid bis(2-ethylhexyl) ester, a salt or a solvate thereof as a first active ingredient and sorbitol as a second active ingredient, wherein sulfosuccinic acid bis(2-ethylhexyl) ester is adsorbed on particles of sorbitol.
In another embodiment, the invention provides fast dissolving granules comprising sulfosuccinic acid bis(2-ethylhexyl) ester, a salt or a solvate thereof as a first active ingredient and sorbitol as a second active ingredient, wherein sulfosuccinic acid bis(2-ethylhexyl) ester is dispersed in particles of sorbitol.
In another embodiment, the invention provides fast dissolving granules comprising sulfosuccinic acid bis(2-ethylhexyl) ester, a salt or a solvate thereof as a first active ingredient and sorbitol as a second active ingredient, wherein sulfosuccinic acid bis(2-ethylhexyl) ester and sorbitol are the sole active ingredients, preferably sulfosuccinic acid bis(2-ethylhexyl) ester, a salt or a solvate thereof, sorbitol and optionally some residual solvent are the sole ingredients.
In another embodiment, the invention provides fast dissolving granules comprising sulfosuccinic acid bis(2-ethylhexyl) ester, a salt or a solvate thereof as a first active ingredient and sorbitol as a second active ingredient, wherein the granules are free from an excipient that is a binder or a disintegrant.
In another embodiment, the invention provides fast dissolving granules comprising sulfosuccinic acid bis(2-ethylhexyl) ester, a salt or a solvate thereof as a first active ingredient and sorbitol as a second active ingredient, wherein the granules are free from a preservative.
In another embodiment, the invention provides fast dissolving granules comprising sulfosuccinic acid bis(2-ethylhexyl) ester, a salt or a solvate thereof as a first active ingredient and sorbitol as a second active ingredient, wherein the granules are free from a benzoate preservative.
In another embodiment, the invention provides fast dissolving granules comprising sulfosuccinic acid bis(2-ethylhexyl) ester, a salt or a solvate thereof as a first active ingredient and sorbitol as a second active ingredient, wherein the granules are free from any excipient.
In another embodiment, the invention provides fast dissolving granules comprising sulfosuccinic acid bis(2-ethylhexyl) ester, a salt or a solvate thereof as a first active ingredient and sorbitol as a second active ingredient, wherein the granules are capable of dissolving in water of 20 °C in less than 10 minutes, measured by dissolving 30 g of granules in 100 ml water.
In another embodiment, the invention provides a pharmaceutical dosage form, comprising fast dissolving granules comprising sulfosuccinic acid bis(2-ethylhexyl) ester, a salt or a solvate thereof as a first active ingredient and sorbitol as a second active ingredient, wherein the dosage form is preferably selected from the group consisting of tablet, capsule, stick pack and sachet.
In another embodiment, the invention provides a pharmaceutical dosage form, comprising fast dissolving granules comprising sulfosuccinic acid bis(2-ethylhexyl) ester, a salt or a solvate thereof as a first active ingredient and sorbitol as a second active ingredient, wherein said dosage form, optionally after reconstitution in water, is suitable for rectal application.
In a further embodiment, the invention provides a kit of parts comprising (a) the fast dissolving granules according to the invention or the pharmaceutical dosage form according to the invention, and (b) a rectal device.
In a further embodiment, the invention provides a kit of parts comprising 30 grams of the granules according to the invention and an enema bottle.
In a still further embodiment, the invention provides a process for preparing fast dissolving granules, comprising sorbitol and sulfosuccinic acid bis(2-ethylhexyl) ester, the process comprising contacting, under agitation, an alcoholic solution of sulfosuccinic acid bis(2-ethylhexyl) ester with sorbitol.

### Detailed description

Accordingly, the invention provides fast dissolving granules comprising sulfosuccinic acid bis(2-ethylhexyl) ester as a first active ingredient and a sugar alcohol as the second active ingredient. Surprisingly, when combined, in the absence of any excipient or any other active ingredient, the particles of the active ingredients form a uniform solid mix having self-binding and self-disintegrating characteristics. Said solid mix is in the form of physically stable granules having a very good blend uniformity preferably in the range of 95 to 105% and having fast dissolving characteristics.

The fast dissolving granules of the invention provide an instant, safe and elegant route for preparation of enema solutions used for bowel evacuation. Apart from eliminating the requirement of potentially unsafe pharmaceutically excipients, granules of the invention allow to instantaneously prepare aqueous solutions of sulfosuccinic acid bis(2-ethylhexyl) ester which is, otherwise, solubilized in water with great difficulty. Further, being in solid, granular form, the pharmaceutical formulation containing the active ingredients has a longer shelf life and is also conveniently transported. Avoiding the use of excipients(s) also contributes to lowering the manufacturing costs of the product, thus allowing better commercial viability. By carefully controlling the preparation in such a way as to obtain maximum dispersion of the active ingredients, optimal use of the ingredients and enhanced process efficiency is ensured. Overall, the manufacturing process is simplified, the ease and safety of use of the product is improved and handling and transport is made convenient, at reduced costs.

When used throughout this specification, the following terms have the meaning indicated:
With the term "fast dissolving granules" it is meant that the granules dissolve within 10 minutes, preferably within 20 seconds to 5 minutes, more preferably within 30 seconds to 3 minutes, when measured by contacting 30 grams of the granules with 100 ml water of 20 °C and manually agitated (shaken) in a container to prepare a solution, free from particles of the granules. The same definition applies to "fast dissolving characteristics" and the like.

Whenever it is stated that the granules are "free from" a certain component, it is meant that said component is substantially absent in the granules, which does not exclude the presence of trace amounts of said component. Preferably, no detectable amount of said component is present in granules free from said component. Likewise, with the term "sole (active) ingredient" it is meant that the granules are free from any other (active) ingredient, as defined above.

The invention provides fast dissolving granules comprising two active ingredients: 1) sulfosuccinic acid bis(2-ethylhexyl) ester and 2) sorbitol. The granules of the invention dissolve rapidly upon contact with water, resulting in a solution that is free from any suspended particles. In the granules, the first active ingredient, namely sulfosuccinic acid bis(2-ethylhexyl) ester, is preferably either adsorbed on the particles of the second active ingredient namely sorbitol or dispersed in the particles of sorbitol. Such an adsorbate or a dispersion can suitably be made by spraying a solution of sulfosuccinic acid bis(2-ethylhexyl) ester over particles of sorbitol, under agitation, resulting in a uniform solid mix.
Thus in one embodiment, the fast dissolving granules according to the invention comprise sulfosuccinic acid bis(2-ethylhexyl) ester, a salt or a solvate thereof that is absorbed on particles of sorbitol.
In one embodiment, the fast dissolving granules according to the invention comprise sulfosuccinic acid bis(2-ethylhexyl) ester, a salt or a solvate thereof that is dispersed in particles of sorbitol.

Solvents that can be used for preparing the solution of the ester include, but are not limited to, ethanol, methanol, propanol, isopropanol, acetone and mixtures thereof.

The process of spraying may conveniently be carried out as follows: A solution of sulfosuccinic acid bis(2-ethylhexyl) ester is sprayed using a peristaltic pump equipped with a spray nozzle over the particles of sorbitol placed in a container. The ester settles over the particles of sorbitol to form a mixture that is subjected to agitation first in a rapid mixer granulator and preferably followed by thorough mixing in a blending machine to form a uniform solid mix of the active ingredients. The solid mix is in the form of physically stable granules having blend uniformity in the range of 95 to 105% and having fast dissolving characteristics. Typically, the granules of the invention dissolve in water of 20 °C within 10 seconds to 10 minutes, preferably within 20 seconds to 5 minutes and more preferably within 30 seconds to 3 minutes.

Blend uniformity is standardly measured using HPLC by dissolving the sample in a solvent mixture and measured against standard samples.

The granules of the invention comprise sulfosuccinic acid bis(2-ethylhexyl) ester and sorbitol as the active ingredients. Though sorbitol is particularly effective in the formulation of the invention, any other polyol or a combination of polyols in amounts in which it is effective as a laxative may be used in the place of sorbitol, provided that it is capable of forming fast dissolving granules in combination with the sulfosuccinic acid bis(2-ethylhexyl) ester. Thus in one embodiment, the invention concerns fast dissolving granules comprising sulfosuccinic acid bis(2-ethylhexyl) ester, a salt or a solvate thereof as a first active ingredient and a polyol, preferably a sugar alcohol as a second active ingredient. Most preferably, the sugar alcohol is sorbitol. Sorbitol can be either in the granular or in the powder form. Preferably, in the granules of the invention, the sulfosuccinic acid bis(2-ethylhexyl) ester is present in an amount of at least 0.2% by weight of polyol, preferably sugar alcohol, most preferably sorbitol. More preferably, the sulfosuccinic acid bis(2-ethylhexyl) ester is present in an amount ranging from 0.2 % by weight to 4 % by weight of the polyol, preferably sugar alcohol, most preferably sorbitol.

In yet another embodiment of the present invention, the granules of the present invention are suitable for reconstitution into an enema solution for rectal use. Importantly, the solution formed from the granules is free from suspended particles and hence provides a homogenous spread of the active ingredients after rectal administration. In the solution that is rapidly formed from the active ingredients, the stool softening attribute of the ester very well combines with the osmotic laxative effect of sorbitol, resulting in a combination effective in bowel evacuation.

In yet another embodiment, the granules of the invention are prepared solely from a mixture of the two active ingredients. The mixture of active ingredients prepared by the process of the invention has been found to have surprisingly effective self-binding and self-disintegrating characteristics, and hence in a preferred embodiment of the present invention, the granules of the invention are formulated without a binder and a disintegrant. Preferably, the granules of the invention are free from any benzoate preservative. The conventionally used benzoate preservatives include, but are not limited to calcium benzoate, sodium benzoate, potassium benzoate and a mixtures thereof. Hence, in a preferred embodiment, the granules according to the invention comprise sulfosuccinic acid bis(2-ethylhexyl) ester, a salt or a solvate thereof and sorbitol as the sole active ingredients, preferably sulfosuccinic acid bis(2-ethylhexyl) ester, a salt or a solvate thereof, sorbitol and optionally residual solvent as the sole ingredients. In one embodiment, the solvent is ethanol, water or both. Thus in one embodiment, the granules according to the invention consist of sulfosuccinic acid bis(2-ethylhexyl) ester, a salt or a solvate thereof, and sorbitol. Preferably the nonsolvent content of the granules according to the invention consist of sulfosuccinic acid bis(2-ethylhexyl) ester, a salt or a solvate thereof and sorbitol. Preferably the solid material of the granules according to the invention consist of sulfosuccinic acid bis(2-ethylhexyl) ester, a salt or a solvate thereof, and sorbitol.

Though the granules of the invention can be prepared without using any pharmaceutical excipient, it can optionally contain various pharmaceutical excipients that are customarily used in preparing pharmaceutical formulations. The pharmaceutical excipients that can optionally be contained in the granules of the invention include lactose, calcium phosphate, microcrystalline cellulose, cellulose powder, kaolin, talc, calcium sulphate, calcium silicate, silica, anti-foaming agents and the like. Preferably, the granules of the invention do not contain any binding agent or a disintegrant such as polyvinylpyrrolidone (PVP, povidone), crosspovidone, hydroxypropylmethylcellulose (HPMC), xanthan gum, croscarmellose sodium, sodium starch glycolate (SSG). Preferably, the granules of the invention are free from any excipient.

The granules of the invention can be used in various solid pharmaceutical dosage forms. The granules can be filled into a mono dose container such as stick packs or sachets or it can be filled in capsules or can be compressed into tablets or caplets. Preferably, the pharmaceutical dosage forms is selected from the group consisting of tablet, capsule stick pack and sachet, more preferably the granules are packaged into a sachet or stick pack.

Preferably, the granules of the present invention are dissolved in aqueous solutions and are administered using a device for rectal administration.

Hence, in yet another aspect of the invention a kit of parts is provided that comprises the granules of the present invention or the pharmaceutical dosage form of the present invention, and a device for rectal administration. Such device can suitably be containers/tools/applicators capable of rectal delivery of an aqueous solution. Examples of such containers/tools/applicators include, but are not limited to enema bottles such as harmonica-type enema bottle, flexible plastic/rubber pouches, flexible plastic/rubber bulbs each of which is preferably equipped with a dispensing means to rectally deliver an aqueous enema solution contained therein. Preferably, the kit of parts comprises the fast dissolving granules of the invention and an enema bottle. In a preferred aspect of the present invention, the kit of parts comprises 30 grams of granules. In an especially preferred embodiment, the kit of part comprises 30 gram of granules in one or more stick packs and an enema bottle of 100 ml capacity.

The invention also discloses a process for preparing fast dissolving granules comprising sorbitol and sulfosuccinic acid bis(2-ethylhexyl) ester. The process of the invention comprises contacting, under agitation, an alcoholic solution of the ester with sorbitol. Preferably , either a granular grade or a powder form of sorbitol is used to prepare the granules of the invention. When sorbitol is used in the powder form, preferably an alcohol/water mixture is used in the spray solution. The sorbitol particles are suitably placed in a container adapted for mixing and the container is agitated at high speeds while the spraying is being carried out. After drying, the mixture is transferred to a blender and agitated again. The overall process renders a uniform solid mix in the form of granules wherein the particles of the ester are uniformly mixed with the particles of sorbitol.

The granules or the dosage form of the invention, optionally after reconstitution into an enema preparation, are useful for the treatment of constipation by stimulating as well as promoting bowel evacuation. An aqueous solution can readily be prepared from the granules or dosage form of the invention and such aqueous solution is preferably administered as enema. The invention is further illustrated by way of the following non-limiting examples.

Blend uniformity of 30 g of representative sample of granules is measured using HPLC by dissolving the sample in a solvent mixture of water and acetonitrile in a molar ratio of 80 : 20 and measured against standard (same concentration as in granules) pure samples of sulfosuccinic acid bis(2-ethylhexyl) ester and sorbitol.

### Example

### Preparation of granules

120 g of sulfosuccinic acid bis(2-ethylhexyl) ester dissolved in 750 ml of ethanol solvent was sprayed over 15.0 kg sorbitol loaded in an RMG (rapid mixer granulator) bowl. The mixture was agitated until granules having optimum consistency (determined by visual examination) of a mixture of the ester and sorbitol were obtained. The granules were then dried. 15.0 kg sorbitol was added and the dried granules were further agitated in the RMG. 8.25 kg sorbitol was additionally added to these granules and the granules were blended in a blending machine to obtain granules containing sulfosuccinic acid bis(2-ethylhexyl) ester and sorbitol.

The granules had blend uniformity in the range of 95 to 105 %. Further, when 30 g of the granules so prepared were agitated manually with 100 ml water of 20 °C in an enema bottle to prepare a particle-free aqueous solution, the granules dissolved in 30 seconds.

## Claims

1. Fast dissolving granules comprising sulfosuccinic acid bis(2-ethylhexyl) ester, a salt or a solvate thereof as a first active ingredient and sorbitol as a second active ingredient, wherein sulfosuccinic acid bis(2- ethylhexyl) ester is present in an amount of 0.2 to 4 % by weight of sorbitol.

2. The granules according to claim 1, wherein sulfosuccinic acid bis(2-ethylhexyl)ester and sorbitol are the sole active ingredients.

3. The granules according to any one of claims 1 to 2, wherein the granules are free from an excipient that is a binder or a disintegrant.

4. The granules according to any one of claim 1 to 3, wherein the granules are free from a preservative.

5. The granules according to claim 4, wherein the preservative is a benzoate preservative.

6. The granules according to any one of claims 1 to 5, wherein the granules are free from any excipient.

7. The granules according to any of claims 1 to 6, wherein 30 g of granules dissolve within 10 minutes in 100 ml of water at 20°C.

8. A pharmaceutical dosage form comprising the granules according to any one of claims 1 to 7, preferably wherein the dosage form is selected from the group consisting of tablet, capsule, stick pack and sachet.

9. The dosage form according to claim 8, which, optionally after reconstitution in water, is suitable for rectal application.

10. A kit of parts comprising (a) the granules according to any one of claims 1 to 7 or the dosage form according to claim 8 or 9 and (b) a rectal device.

11. The kit of parts according to claim 10, wherein the granules are present in an amount of 30 grams and the device is an enema bottle.

12. A process for preparing fast dissolving granules comprising sorbitol and sulfosuccinic acid bis(2-ethylhexyl) ester, comprising contacting, under agitation, an alcoholic solution of sulfosuccinic acid bis(2-ethylhexyl) ester with sorbitol.

## Patentansprüche

1. Schnelllösliche Granalien, umfassend Sulfobernsteinsäure-bis(2-ethylhexyl)ester, ein Salz oder ein Solvat davon als ersten Wirkstoff und Sorbit als zweiten Wirkstoff, worin Sulfobernsteinsäure-bis(2-ethylhexyl)ester in einer Menge von 0,2 bis 4 Gewichtsprozent bezogen auf Sorbit vorhanden ist.

2. Granalien gemäß Anspruch 1, worin Sulfobernsteinsäurebis(2-ethylhexyl)ester und Sorbit die einzigen Wirkstoffe sind.

3. Granalien gemäß einem der Ansprüche 1 bis 2, worin die Granalien frei von Hilfsstoff sind, der ein Bindemittel oder ein Sprengmittel ist.

4. Granalien gemäß einem der Ansprüche 1 bis 3, worin die Granalien frei von Konservierungsmitteln sind.

5. Granalien gemäß Anspruch 4, worin das Konservierungsmittel ein Benzoat-Konservierungsmittel ist.

6. Granalien gemäß einem der Ansprüche 1 bis 5, worin die Granalien frei von Hilfsstoff sind.

7. Granalien gemäß einem der Ansprüche 1 bis 6, worin sich 30 g Granalien innerhalb von 10 Minuten in 100 ml Wasser bei 20 °C auflösen.

8. Pharmazeutische Arzneiform, die die Granalien gemäß einem der Ansprüche 1 bis 7 umfasst, worin die Arzneiform vorzugsweise aus der Gruppe ausgewählt ist, die aus einer Tablette, einer Kapsel, einem Stick Pack und einem Portionsbeutel besteht.

9. Arzneiform gemäß Anspruch 8, die, gegebenenfalls nach Wiederherstellung in Wasser, zur rektalen Anwendung geeignet ist.

10. Bausatz, der (a) die Granalien gemäß einem der Ansprüche 1 bis 7 oder die Arzneiform gemäß Anspruch 8 oder 9 und (b) eine Rektalvorrichtung umfasst.

11. Bausatz gemäß Anspruch 10, worin die Granalien in einer Menge von 30 g vorhanden sind und die Vorrichtung eine Klistierflasche ist.

12. Verfahren zur Herstellung schnelllöslicher Granalien, die Sorbit und Sulfobernsteinsäure-bis(2-ethylhexyl)ester umfassen, umfassend das Inkontaktbringen einer alkoholischen Lösung von Sulfobernsteinsäure-bis(2-ethylhexyl)ester unter Rühren mit Sorbit.

## Revendications

1. Granules à dissolution rapide comprenant du sulfosuccinate de di (éthyl-2-hexyle), un sel ou un solvate de celui-ci en tant que premier principe actif et du sorbitol en tant que second principe actif, dans lesquels le sulfosuccinate de di (éthyl-2- hexyle) est présent en une quantité de 0,2 à 4 % en poids de sorbitol.

2. Granules selon la revendication 1, dans lesquels le sulfosuccinate de di (éthyl-2-hexyle) et le sorbitol sont les seuls principes actifs.

3. Granules selon l'une quelconque des revendications 1 à 2, dans lesquels les granules sont exempts d'excipient qui est un liant ou un délitant.

4. Granules selon l'une quelconque des revendications 1 à 3, dans lesquels les granules sont exempts de conservateur.

5. Granules selon la revendication 4, dans lesquels le conservateur est un conservateur benzoate.

6. Granules selon l'une quelconque des revendications 1 à 5, dans lesquels les granules sont exempts d'excipient.

7. Granules selon l'une quelconque des revendications 1 à 6, dans lesquels 30 g de granules sont dissous en 10 minutes dans 100 ml d'eau à 20 °C.

8. Forme posologique pharmaceutique comprenant les granules selon l'une quelconque des revendications 1 à 7, de préférence la forme posologique étant choisie dans le groupe constitué par un comprimé, une gélule, un sachet tubulaire et un sachet.

9. Forme posologique selon la revendication 8, qui, facultativement après la reconstitution dans l'eau, est appropriée pour une application rectale.

10. Kit d'éléments comprenant (a) les granules selon l'une quelconque des revendications 1 à 7 ou la forme posologique selon la revendication 8 ou 9 et (b) un dispositif rectal.

11. Kit d'éléments selon la revendication 10, dans lequel les granules sont présents en une quantité de 30 g et le dispositif est une bouteille de lavement.

12. Procédé de préparation de granules à dissolution rapide comprenant du sorbitol et du sulfosuccinate de di (éthyl-2-hexyle), comprenant la mise en contact, sous agitation, d'une solution alcoolique du sulfosuccinate de di (éthyl-2-hexyle) avec du sorbitol.
